# EUROPEAN PATENT APPLICATION

(11) **EP 2 305 258 A2**
(43) Date of publication of application: **06.04.2011**
(21) Application number: 10012090.6
(22) Date of filing: 31.05.2006
(51) Int. Cl.: A61K 31/4436, A61K 38/17, A61P 25/00

(54) **Inhibition of neuronal damage**

(30) Priority: 31.05.2005 US 685802 P
(62) Divisional of application: 06771718.1
(71) Applicant: Duska Scientific Co, Bala Cynwyd, PA 19004 (US)
(72) Inventor: Galli, Lucia, I-35020 Polverara Padova (IT); Resta, Valentina, I-56100 Pisa (IT); Divirgilio, Francesco, I-56019 Vecchiano Pisa (IT)
(74) Representative: White, Martin Paul

(57) **Abstract**

Materials and methods for inhibiting neuronal damage are provided herein. In particular, materials and methods for inhibiting neuronal damage associated with pathological action of extracellular ATP are provided herein.

## Description

### CROSS-REFERENCE TO RELATED APPLICATIONS

This application claims priority from U.S. Provisional Application Serial No. 60/685,802, filed May 31, 2005.

### TECHNICAL FIELD

This document relates to materials and methods for blocking neuronal damage, and in particular to materials and methods for blocking neuronal damage associated with pathological action of ATP.

### BACKGROUND

Nerve damage is a debilitating result of a number of clinical conditions and diseases. Increased extracellular pressure is a major damaging factor in several diseases of the central nervous system and in the course of surgery of the eye and of the brain. In particular, increased intraocular pressure (IOP) is a major risk factor for retinal ganglion cell (RGC) injury, as it is observed in glaucoma, a family of retinal diseases characterized by progressive loss of RGCs and a leading cause of blindness (Collaborative, normal-tension, glaucoma, study and group (1998) Am. J. Ophthalmol. 126:487-497). The mechanisms of pressure-induced RGC damage are unclear (Quigley (1999) Prog. Retin. Eye Res. 18:39-57). New therapeutic modalities are needed for treatment and/or prevention of neuronal cell damage associated with increased pressure that is observed with injuries, surgeries, and pathophysiological conditions such as glaucoma.

### SUMMARY

This document is based in part on the discoveries that increased IOP can lead to release of adenosine 5'-triphosphate (ATP) into the eye fluid, and that application of extracellular ATP to isolated retinas can mimic pressure-induced RGC damage. This document also is based in part on the discovery that agents that degrade ATP, or antagonists of ATP's effect on adenosine receptors, can be used to inhibit pressure-induced RGC damage. Thus, methods are described herein to protect neurons from damage by blocking the pathologic action of extracellular ATP (eATP). These methods can include, for example, administration (e.g., by intraocular injection) of agents that degrade ATP (such as apyrase) or antagonists of the P2X receptors (*e*.*g*., the P2X₇ receptor). Such methods may be useful in treatment of conditions such as glaucoma, or for reducing nerve damage that can result from injury or surgery.

In one aspect, this document features a method for inhibiting neuronal damage. The method can include (a) identifying a mammalian subject that has, is likely to have, or is likely to develop neuronal damage; and (b) delivering to a neuron in the subject, or to the extracellular environment of said neuron, one or more agents that inhibit the pathologic action of ATP on neurons. The mammalian subject can be a human. The neuronal damage can be due to increased extracellular pressure. The increased extracellular pressure can be spontaneous, traumatic, pathologic, or associated with surgery. The increased extracellular pressure can be intraocular, intracranial, or in the spinal fluid. The neuron can be a retinal neuron (e.g., a retinal ganglion cell). The neuron can be a cortical neuron, a hippocampal neuron, a basal ganglia, or a spinal cord neuron. The agent can be an apyrase. The agent can be an inhibitor of a P2X receptor (e.g. , a P2X₇ receptor). The agent can be Brilliant BlueG, oxidized-ATP, an antibody or an antibody fragment that binds specifically to a P2X receptor, a P2X receptor mRNA-specific oligonucleotide, a P2X receptor small interfering RNA, or a P2X nucleic acid aptamer.

In another aspect, this document features a composition containing a pharmaceutically acceptable carrier and an agent that inhibits the pathologic action of ATP on neurons. This document also features an article of manufacture containing packaging material, one or more agents that inhibit the pathologic action of ATP on neurons, and written instructions for use of the one or more agents in the methods described herein. In addition, this document features a kit containing two or more agents that inhibit the pathologic action of ATP on neurons.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF DRAWINGS

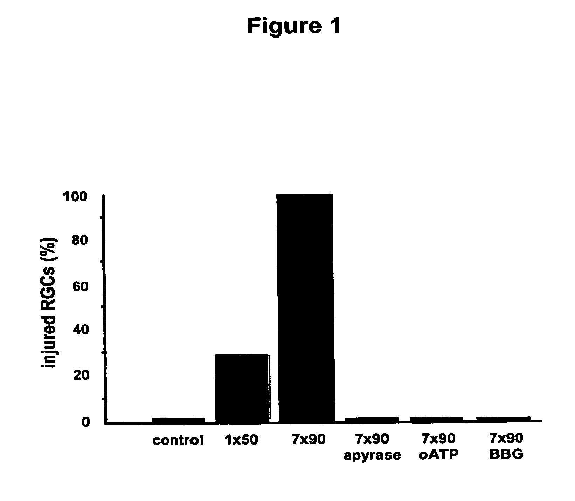

FIG 1 is a graph showing quantification of pressure effects on non-α-type RGCs in the presence or absence of agents that block eATP signaling. Control = 1 hour at 12 mm Hg, N = 40 RGCs; 1x50 = 1 minute at 50 mm Hg followed by 1 hour at 12 mm Hg, N = 45 RGCs; 7x90 = 7 consecutive 1 minute exposures to 90 mm Hg followed by 1 hour at 12 mm Hg, N = 47 RGCs.

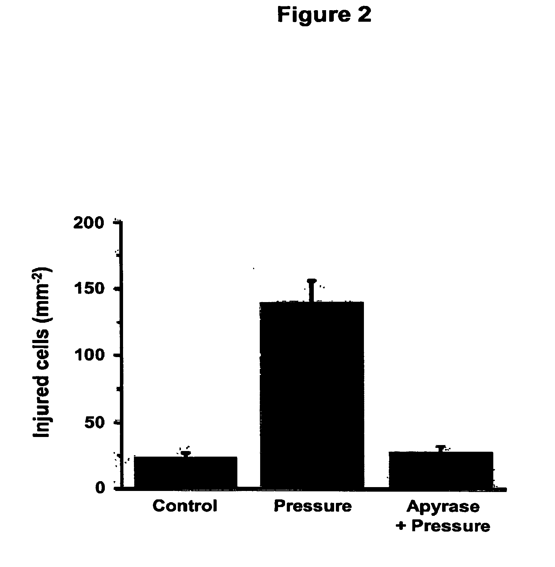

FIG 2 is a graph showing quantification of PI staining of cells in the ganglion cell layer after a pressure pulse *in vivo,* or after a pressure pulse in the presence of apyrase. P < 10⁻⁵; N=8 retinas for each experimental condition.

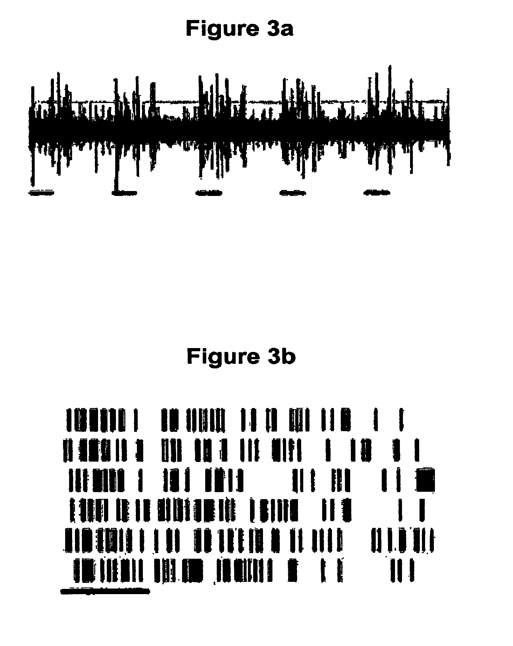

FIG 3a is a representative recording of extracellular activity in the optic tract, showing multiunit responses to a series of light flashes. Horizontal bars under the trace represent light ON periods (duration of each flash = 1.25 sec). The continuous line placed on the trace exemplifies qualitatively the thresholding procedure for spike discrimination. FIG 3b is a raster display showing examples of spike recordings in response to 6 consecutive light flashes. Each vertical bar on the raster plot represents an extracellularly recorded action potential. Each row is a the response to a single flash. The horizontal bar under the trace indicates flash duration (= 1.25 sec). The top row corresponds to the first flash of the series.

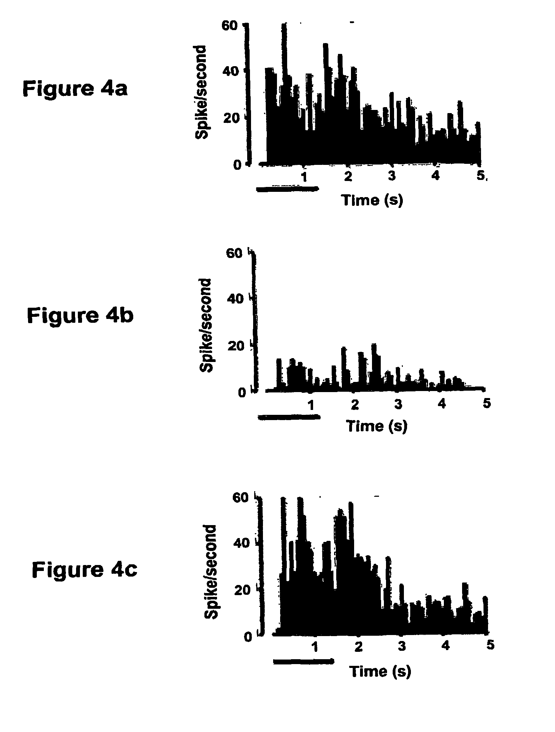

FIGS. 4a, 4b, and 4c are examples of peristimulus time histograms (PSTH) from multi-unit spike activity recorded in the optic tract in response to a 1.25 sec flash of light right before (c), 5 minutes after (d), and 40 minutes after (e) an IOP transient of 1 minute to 90 mmHg. Spike responses were obtained by computing the firing rate every 78 ms, and averaging over 10 consecutive stimulations. The bars underneath each graph represent flash duration.

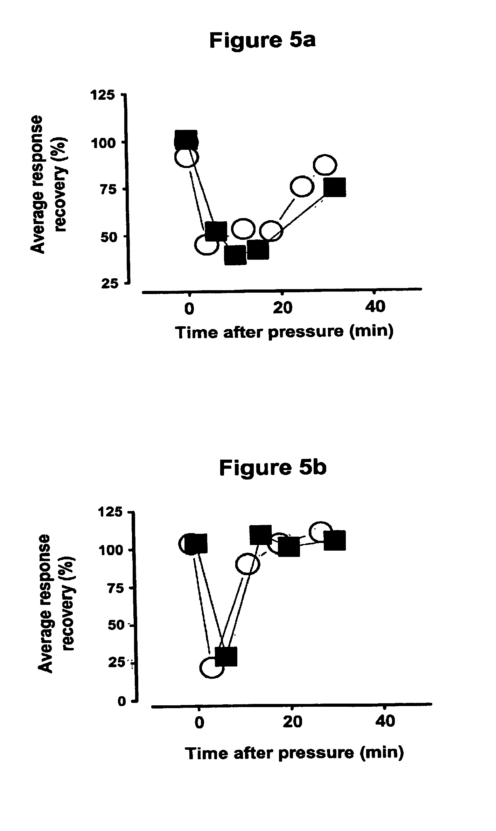

FIG 5a shows a time-course of response recovery after 1 minute IOP transient to 90 mmHg in control animals. Symbol size is larger than error bars. Average response was quantified as the average spike activity in the 0-2 sec time interval of each PSTH. FIG 5b shows a time-course of response recovery after 1 minute IOP transient to 90 mmHg in animals treated by intraocular injection of apyrase prior to IOP increments.

### DETAILED DESCRIPTION

ATP is a purine nucleotide found in every living cell, where it plays a critical role in cellular metabolism and energetics. ATP is released from cells under physiologic and pathophysiologic conditions; extracellular ATP acts as a local physiologic regulator as well as an endogenous mediator that plays a mechanistic role in the pathophysiology of obstructive airway diseases, for example (Pelleg et αl. (2002) Am. J. Ther. 9:454-464). In addition, ATP exerts potent effects on dendritic cells, eosinophils and mast cells. For example, ATP enhances IgE-mediated release of histamine and other mediators from human lung mast cells (Schulman et al. (1999) Am. J. Respir. Cell. Mol. Biol. 20:530-537).

Extracellular ATP affects many cell types in different tissues and organs by activating cell surface receptors known as P2 purinergic receptors (P2R). P2R are divided into two families: P2X, which are ligand-binding, dimeric, trans-cell membrane cationic channels, and P2Y, which are seven trans-cell membrane domain G protein-coupled receptors. Eight P2Y (P2Y₁, P2Y₂, P2Y₄, P2Y₆, P2Y₁₁, P2Y₁₂, P2Y₁₃, and P2Y₁₄), seven homodimeric P2X receptor subtypes (P2X)₁₋₇), and five P2X heterodimeric receptors (X_{1/2}, X_{2/3}, X_{2/6}, X_{1/5}, and X_{1/6}) have been identified and cloned.

Increased extracellular fluid pressure (e.g., intraocular, intracranial, or spinal) is a major damaging factor in several diseases of the central nervous system and in eye and brain surgery. Increased IOP can lead to release of ATP into eye fluid. As described herein, application of extracellular ATP to isolated retinas can mimic pressure-induced RGC damage. Thus, methods are provided herein to protect neurons from damage caused by pathologic action of ATP. As described herein, these methods can include administration of agents that block the pathologic action of ATP. Such agents can include, for example, apyrase and other agents that can degrade ATP, or antagonists of P2X receptors (e.g., the P2X₇ ATP receptor). The methods provided herein may be useful in treatment of clinical conditions (*e*.*g*., glaucoma), or for reducing neural damage that can result from injury or surgery.

Neurons that can be protected using the methods provided herein can include those in nerves of the central nervous system (CNS) (*e*.*g*., brain or spinal cord neurons, or RGC) as well as the peripheral nervous system (PNS) (e.g., autonomic, spinal, or cranial nerves). Nerve damage that in general is related to pathologic action of ATP can include, for example, hydrocephaly, edema, mechanical and compressive traumas, excitotoxic damage, and ischemia.

### 1. Agents that can inhibit the pathologic αction of ATP

The methods provided herein can include administration of agents that inhibit or block the pathologic action of ATP. Such agents can include, for example, agents that degrade ATP, antagonists of P2X ATP receptors such as the P2X₇ and P2X₄ receptors, and agents that reduce or prevent expression of proteins involved in ATP synthesis or in ATP signalling.

Examples of agents that can degrade ATP include, without limitation, ecto-nucleotidases including apyrases such as ecto-ATPase and CD39 (also known as ecto-ATP diphosphohydrolase, NTP-Dase-1, or ecto-apyrase), hexokinase, and other enzymes that hydrolyze extracellular ATP.

As used herein, the term "P2X receptor antagonist" includes agents that (a) inhibit activation by a P2X receptor agonist of cells expressing a P2X receptor; or (b) inhibit the activity of a cell expressing a P2X receptor. Such P2X receptor antagonists can act by completely or substantially inhibiting binding of an agonist to a P2X receptor by binding to the binding site of the relevant agonist on the receptor, or they can act allosterically by binding at a site other than the agonist binding site and inducing a conformational change in the P2X receptor such that binding of an agonist to the receptor is substantially, if not completely, inhibited. Alternatively, a P2X receptor antagonist can inhibit an activity of a cell expressing a P2X receptor by binding to the receptor, either at an agonist-binding site or at a separate site, and delivering an inhibitory signal to the cell. Further, P2X receptor antagonists can act at sites downstream from the P2X receptor by interfering with one or more steps of the relevant signal transduction initiated by the receptor.

Examples of antagonists of P2X receptors include, without limitation, Brilliant BlueG, a selective and reversible inhibitor of rat P2X₇ receptors; oxidized-ATP (oATP), an irreversible blocker of P2X receptors; pyridoxalphosphate-6-azophenyl-2',4'-disulphonic acid•4Na (PPADS), a functionally selective P2X purinoceptor antagonist; suramine, an unspecific P2X receptor antagonist; 1-[N,O-bis(5-isoquinolinesulfonyl)-N-methyl-L-tyrosyl]-4-phenylpiperazine (KN-62), a selective inhibitor of rat brain Ca⁺²/calmodulin-dependent protein kinase II; and 5-(N,N-hexamethylene)amiloride (HMA), an inhibitor of Na⁺/H⁺ antiport, as well as 5-{[3"-diphenylether (1',2',3',4'-tetrahydronaphthalen-1-yl)amino]carbonyl}benzene-1, 2, 4-tricarboxylic acid; 2',3'-*O-*(4-benzoylbenzoyl)-ATP (BzATP); tetramethylpyrazine (TMP); and 2',3'-*O*-2,4,6-trinitrophenyl-ATP (TNP-ATP). Additional examples of P2X receptor antagonists include those listed in U.S. Patent No. 6,831,193, which is incorporated herein by reference in its entirety.

Small molecule compounds known as nucleic acid aptamers also can be used to reduce or block pathologic activity of ATP. Nucleic acid aptamers are relatively short nucleic acid (DNA, RNA or a combination of both) molecules that can bind with high avidity to proteins (e.g., any of the P2X receptors listed herein) and inhibit the binding to such proteins of ligands, receptors, and other molecules. Aptamers generally are about 25 to 40 nucleotides in length and can have molecular weights in the range of about 18 to 25 kDa. Aptamers with high specificity and affinity for targets can be obtained using an *in vitro* evolutionary process termed SELEX (systemic evolution of ligands by exponential enrichment) (see, for example, Zhang et al. (2004) Arch. Immunol. Ther. Exp. 52:307-315). For methods of enhancing the stability (using nucleotide analogs, for example) and *in vivo* bioavailability (*e.g., in vivo* persistence in a subject's circulatory system) of nucleic acid aptamers, see Zhang *et αl.* (*suprα*) and Brody et αl. (2000) Rev Mol. Biotechnol. 74:5-13.

In addition, non-agonist antibodies having specific binding affinity for the P2X receptors (e.g., the P2X₇ receptor) or other molecules involved in ATP signalling can be used to reduce the pathologic activity of ATP. To produce antibodies, host animals such as rabbits, chickens, mice, guinea pigs, or rats can be immunized by injection of a target polypeptide or an antigenic fragment of a target polypeptide (e.g., a P2X receptor such as P2X₇). Various adjuvants can be used to increase the immunological response, depending on the host species. These include Freund's adjuvant (complete and incomplete), mineral gels such as aluminum hydroxide, surface active substances such as lysolecithin, pluronic polyols, polyanions, peptides, oil emulsions, keyhole limpet hemocyanin, and dinitrophenol. The antibodies can be polyclonal or monoclonal. Polyclonal antibodies are heterogeneous populations of antibody molecules that are contained in the sera of the immunized animals. Monoclonal antibodies, which are homogeneous populations of antibodies to a particular antigen, can be prepared using a target polypeptide (or an antigenic fragment thereof) and standard hybridoma technology. In particular, monoclonal antibodies can be obtained using any technique that provides for the production of antibody molecules by continuous cell lines in culture such as described by Kohler et αl., Nature, 256:495 (1975), the human B-cell hybridoma technique (Kosbor et αl., Immunology Today, 4:72 (1983); Cole et al., Proc. Nαtl. Acαd. Sci USA, 80:2026 (1983)), and the EBV-hybridoma technique (Cole et αl., Monoclonal Antibodies and Cancer Therapy, Alan R. Liss, Inc., pp. 77-96 (1983). Such antibodies can be of any immunoglobulin class, including IgG, IgM, IgE, IgA, IgD, and any subclass thereof. A hybridoma producing a monoclonal antibody can be cultivated *in vitro* or *in vivo.* Antibodies can be made in or derived from any of a variety of species, e.g., humans, non-human primates (e.g., monkeys, baboons, or chimpanzees), horses, cattle, pigs, sheep, goats, dogs, cats, rabbits, guinea pigs, gerbils, hamsters, rats, and mice.

Antibody fragments that have specific binding affinity for a target polypeptide also can be useful, and can be generated using known techniques. As used herein, the term "antibody fragment" refers to an antigen-binding fragment, e.g., Fab, F(ab')₂. Fv, and single chain Fv (scFv) fragments. F(ab')2 fragments can be produced by pepsin digestion of an antibody molecule, and Fab fragments can be generated by reducing the disulfide bridges of F(ab')2 fragments. Alternatively, Fab expression libraries can be constructed. See, for example, Huse et αl., Science, 246:1275 (1989). Once produced, antibodies or fragments thereof are tested for recognition of PNMT variant polypeptides by standard immunoassay methods including ELISA techniques, radioimmunoassays and Western blotting. See, Short Protocols in Molecular Biology, Chapter 11, Green Publishing Associates and John Wiley & Sons, edited by Ausubel et αl., 1992. An scFv fragment is a single polypeptide chain that includes both the heavy and light chain variable regions of the antibody from which the scFv is derived. scFv fragments can be produced, for example, as described in U.S. Patent No. 4,642,334. In addition, diabodies [Poljak (1994) Structure 2(12):1121-1123; Hudson et αl. (1999) J. Immunol. Methods 23(1-2):177-189] and intrabodies [Huston et αl. (2001) Hum. Antibodies 10(3-4):127-142; Wheeler et αl. (2003) Mol. Ther. 8(3):355-366; Stocks (2004) Drug Discov. Today 9(22): 960-966] can be used in the methods provided herein.

An antibody can be a purified or a recombinant antibody. Also useful are chimeric antibodies and humanized antibodies made from non-human (e.g., mouse, rat, gerbil, or hamster) antibodies. Chimeric and humanized monoclonal antibodies can be produced using recombinant DNA techniques known in the art, for example, using methods described in International Patent Publication PCT/US86/02269; European Patent Application 184,187; European Patent Application 171,496; European Patent Application 173,494; PCT Application WO 86/01533; U.S. Patent No. 4,816,567; European Patent Application 125,023; Better et αl. (1988) Science 240:1041-1043; Liu et αl. (1987) J. Immunol. 139:3521-3526; Sun et αl. (1987) Proc. Nαtl. Acαd Sci. USA 84:214-218; Nishimura et αl. (1987) Cαnc. Res. 47:999-1005; Wood et αl. (1985) Nature 314:446-449; Shaw et αl. (1988) J. Nαtl. Cancer Inst. 80:1553-1559; Morrison (1985) Science 229:1202-1207; Oi et αl. (1986) BioTechniques 4:214; U.S. Patent No. 5,225,539; Jones et αl. (1986) Nature 321:552-525; Veroeyan et αl. (1988) Science 239:1534; and Beidler et αl. (1988) J. Immunol. 141:4053-4060.

Agents that can be used to inhibit expression of proteins involved in ATP synthesis or in ATP signaling can include antisense nucleic acids (e.g., oligonucleotides) and interfering RNAs. Antisense compounds generally are used to interfere with protein expression by, for example, interfering directly with translation of a target mRNA molecule, by RNAse-H-mediated degradation of the target mRNA, by interference with 5' capping of mRNA, by preventing translation factor binding to the target mRNA by masking of the 5' cap, or by inhibiting mRNA polyadenylation. The interference with protein expression arises from the hybridization of the antisense compound with its target mRNA. A specific targeting site on a target mRNA of interest for interaction with an antisense compound is chosen. Thus, for example, for modulation of polyadenylation, a target site on an mRNA target can be a polyadenylation signal or a polyadenylation site. For diminishing mRNA stability or degradation, destabilizing sequences are useful as target sites. Once one or more target sites have been identified, oligonucleotides can be chosen which are sufficiently complementary to the target site (*i*.*e*., hybridize sufficiently well under physiological conditions and with sufficient specificity) to give the desired effect.

The term "oligonucleotide" as used herein refers to an oligomer or polymer of RNA, DNA, or a mimetic of either. The term includes oligonucleotides composed of naturally-occurring nucleobases, sugars, and covalent internucleoside (backbone) linkages. The normal linkage or backbone of RNA and DNA is a 3' to 5' phosphodiester bond. The term also refers, however, to oligonucleotides composed entirely of, or having portions containing, non-naturally occurring components that function in a similar manner to the oligonucleotides containing only naturally-occurring components. Such modified substituted oligonucleotides may be preferred over native forms because of desirable properties such as, for example, enhanced cellular uptake, enhanced affinity for target sequence, and increased stability in the presence of nucleases. In the mimetics, the core base (pyrimidine or purine) structure is generally preserved but (1) the sugars are either modified or replaced with other components and/or (2) the inter-nucleobase linkages are modified. One class of nucleic acid mimetic that can be very useful is referred to as protein nucleic acid (PNA). In PNA molecules, the sugar backbone is replaced with an amide-containing backbone (*e*.*g*., an aminoethylglycine backbone). The bases are retained and are bound directly to aza nitrogen atoms of the amide portion of the backbone. PNAs and other mimetics that may be useful in the methods disclosed herein are described in detail in U.S. Patent No. 6,210,289.

Antisense oligomers that can be used in the methods provided herein generally can contain about 8 to about 100 (*e*.*g*., about 14 to about 80 or about 14 to about 35) nucleobases (or nucleosides where the nucleobases are naturally occurring).

One or more antisense oligonucleotides can themselves be introduced into a cell to reduce or prevent expression of a particular protein (e.g., any of the P2X receptors listed herein). Alternatively, an expression vector containing a nucleic sequence encoding the antisense oligonucleotide can be introduced into the cell. In the latter case, the oligonucleotide produced by the expression vector is an RNA oligonucleotide composed entirely of naturally occurring components. The sequence encoding the RNA oligonucleotide can be operably linked to a promoter to drive transcription of the oligonucleotide. As used herein, "operably linked" means incorporated into a genetic construct so that one or more expression control sequences can effectively control expression of a coding sequence of interest. A coding sequence is "operably linked" and "under the control" of an expression control sequence in a cell when RNA polymerase is able to transcribe the coding sequence into mRNA.

Enhancers provide expression specificity in terms of time, location, and level. Unlike a promoter, an enhancer can function when located at variable distances from the transcription initiation site, provided a promoter is present. An enhancer can also be located downstream of the transcription initiation site. To bring a coding sequence under the control of a promoter, it is necessary to position the translation initiation site of the translational reading frame of the peptide or polypeptide between one and about fifty nucleotides downstream (3') of the promoter. Promoters of interest can include, without limitation, the cytomegalovirus hCMV immediate early gene, the early or late promoters of SV40 adenovirus, the lac system, the trp system, the TAC system, the TRC system, the major operator and promoter regions of phage A, the control regions of fd coat protein, the promoter for 3-phosphoglycerate kinase, the promoters of acid phosphatase, and the promoters of the yeast α-mating factors, the adenoviral Elb minimal promoter, or the thymidine kinase minimal promoter. The coding sequence of the expression vector is operatively linked to a transcription terminating region.

Suitable expression vectors include plasmids and viral vectors such as herpes viruses, retroviruses, vaccinia viruses, attenuated vaccinia viruses, canary pox viruses, adenoviruses and adeno-associated viruses, among others.

Double-stranded small interfering RNA (siRNA) homologous to a particular DNA also can be used to reduce expression of that DNA. For example, siRNA homologous to a P2X receptor (*e*.*g*., P2X₇) DNA can also be used to reduce expression of P2X₇ in neural cells. See, e.g., Fire et αl. (1998) Nature 391:806-811; Romano and Masino (1992) Mol. Microbiol. 6:3343-3353; Cogoni et αl. (1996) EMBO J. 15:3153-3163; Cogoni and Masino (1999) Nature 399:166-169; Misquitta and Paterson (1999) Proc. Natl. Acad Sci. USA 96:1451-1456; and Kennerdell and Carthew (1998) Cell 95:1017-1026.

The sense and anti-sense RNA strands of siRNA can be individually constructed using chemical synthesis and enzymatic ligation reactions using procedures known in the art. For example, each strand can be chemically synthesized using naturally occurring nucleotides or variously modified nucleotides designed to increase the biological stability of the molecule or to increase the physical stability of the duplex formed between the sense and anti-sense strands, e.g., phosphorothioate derivatives and acridine substituted nucleotides. The sense or anti-sense strand can also be produced biologically using an expression vector into which a target sequence (full-length or a fragment) has been subcloned in a sense or anti-sense orientation. The sense and anti-sense RNA strands can be annealed *in vitro* before delivery of the dsRNA to cells. Alternatively, annealing can occur *in vivo* after the sense and anti-sense strands are sequentially delivered to neural cells.

Double-stranded siRNA interference can be achieved by introducing into a neural cell a polynucleotide from which sense and anti-sense RNAs can be transcribed under the direction of separate promoters, or a single RNA molecule from which both sense and anti-sense sequences can be transcribed under the direction of a single promoter.

It is possible that extracellular ATP acts directly on neurons (e.g., RGCs) to cause their death. However, it also is possible that extracellular ATP also acts on bystander cells (e.g., glial cells in the retina) to promote the release of toxic factors *(e.g.,* NO or superoxide anion, or glutamate) that can synergise with ATP in causing damage of RGCs. Thus, in addition to the above-described agents, it is possible that other agents may be useful to reduce or prevent damage derived from ATP-mediated activation of sudibystander glial cells. Such agents can include, for example, tumor necrosis factor-α (TNFα) and interleukin 1-β (IL-1β).

### 2. Methods

The agents described herein can be delivered *in vitro* or *in vivo. In vitro* methods can be useful as, for example, "positive controls" in screening assays to test the effectiveness of particular agents or to optimize survival and/or growth of neurons in cultures set up to produce neurons for any of a variety of purposes, e.g., drug screening, toxicity testing, etc. Such methods can include administering to a neural cell *in vitro* one or more agents that can reduce or inhibit the pathologic action of ATP on neurons. These agents are referred to herein as "ATP inhibitory agents."

The *in vivo* methods provided herein can be used to inhibit neuronal damage in a subject. The methods can include delivering to a neuron in the subject, or to the extracellular environment of the neuron, one or more agents that inhibit the pathologic action of ATP on neurons. Prior to the delivering, the methods also can include the step of identifying a subject that has, is likely to have, or is likely to develop neuronal damage (e.g., neuronal damage related to pressure, as can occur with glaucoma, for example). Alternatively, one or more agents that inhibit the production of substances that are toxic to neurons by bystander cells responding to ATP can be delivered to such a bystander cell or to the extracellular environment of such a bystander cell.

The methods provided herein can be therapeutic or prophylactic. As used herein, an agent that is "therapeutic" is an agent that causes a complete abolishment of the symptoms of a disease or condition, or a decrease in the severity of the symptoms of the disease or condition. "Prevention" means that symptoms of the disease or condition are essentially absent. As used herein, "prophylaxis" means complete prevention of the symptoms of a disease or condition, a delay in onset of the symptoms of a disease or condition, or a lessening in the severity of subsequently developed symptoms. In some embodiments, the methods provided herein can include monitoring the subject for neural damage (e.g., a reduction in neural damage or for the presence/absence of neural damage) during or after treatment with one or more inhibitory agents as described herein and, if desired, delivering another dose of the same agent or a dose of a different agent to the neuron or the extracellular environment of the neuron.

A "subject" can be any mammalian subject. For example, a subject can be a rat, mouse, dog, cat, rabbit, horse, bovine (*e*.*g*., cow, bull, or ox), goat, sheep, pig, non-human primate (e.g. , chimpanzee or orangutan), or human. By "has, is likely to have, or is likely to develop" is meant that the subject has been diagnosed with or is at risk for having or developing neuronal damage. For example, a subject that will undergo a particular type of surgery (e.g., ocular surgery) may be likely to develop neuronal damage. Similarly, a subject at risk for glaucoma may be considered likely to have or develop neuronal damage.

The neurons subject to increased pressure can be any neuron of the CNS or PNS, as described herein. By "increased pressure" is meant extracellular pressure that is sufficiently increased to cause neuronal damage, and generally can be at least 10% (e.g., 10%, 15%, 20%, 25%, 30%, 35%, 40%, 45%, 50%, 55%, 60%, 65%, 70%, 75%, 80%, 85%, 90%, 95%, 100%, or more than 100%) greater than the normal, ambient extracellular pressure to which a neuron is typically subjected. Such increased pressure can result from conditions such as, without limitation, glaucoma, intracranial surgery, neural trauma, ophthalmic surgery, brain edema, and hydrocephaly.

The term "extracellular environment" as used herein refers to the environment immediately surrounding a cell. The extracellular environment of a particular cell can include, for example, any extracellular matrix or fluid adjacent to the cell, such that, when an ATP inhibitory agent is delivered to the extracellular environment of a neuron or a bystander cell, the agent can either bind to the surface of or enter the neuron or bystander cell, respectively.

The phrase "pathologic action of ATP" refers to adverse effects on a cell (e.g., a neural cell) that result from or are related to ATP signalling. Pathologic action of ATP on neurons can result, for example, in cell death or in impaired cell function.

The term "inhibit" as used herein with reference to neuronal damage, refers to any reduction in neuronal damage by an ATP inhibitory agent. In some embodiments, inhibition of neuronal damage can be as little as a 5% reduction in neural damage as compared to a corresponding untreated neuron, and can be as much as complete (i.e., 100%) inhibition of neural damage as compared to a corresponding untreated neuron. Thus, "inhibiting" neural damage encompasses a reduction of neural damage in a treated neuron of between 5% and 100% (*e*.*g*., 5%, 10%, 15%, 20%, 25%, 30%, 35%, 40%, 50%, 55%, 60%, 65%, 70%, 75%, 80%, 85%, 90%, 95%, 98%, 99%, or 100%) as compared to a corresponding untreated neuron. In some embodiments, reduction in neural damage can be a reduction in the number of damaged neurons in a treated subject or a treated eye versus an untreated subject or an untreated eye. Thus, "inhibiting" neural damage can encompass a reduction of between 5% and 100% (*e*.*g*., 5%, 10%, 15%, 20%, 25%, 30%, 35%, 40%, 50%, 55%, 60%, 65%, 70%, 75%, 80%, 85%, 90%, 95%, 98%, 99%, or 100%) in the number of damaged neurons in a treated subject or a treated eye as compared to an untreated subject or an untreated eye.

Generally, the ATP inhibitory agents useful in methods provided herein can be suspended in a pharmaceutically-acceptable carrier and administered to a subject in a therapeutically effective amount. Pharmaceutically acceptable carriers are biologically compatible vehicles that are suitable for administration to a human (e.g., physiological saline or liposomes). A "therapeutically effective amount" is an amount of an agent that is capable of producing a medically desirable result (*e*.*g*., reduced pathologic action of ATP on neurons) in a treated subject. The agents can be administered via any suitable route. For example, one or more agents can be administered to a subject orally or injected intravenously, subcutaneously, intramuscularly, intrathecally, intraperitoneally, intrarectally, intravaginally, intranasally, intragastrically, intratracheally, or intrapulmonarily. They can also be delivered directly to neural cells, e.g., intraocularly delivered to the retina. The dosage required depends on the choice of the route of administration the nature of the formulation, the nature of the patient's illness, the subject's size, weight, surface area, age, and sex, other drugs being administered, and the judgment of the attending clinician. Suitable dosages can be in the range of 0.0001 mg/kg to 100 mg/kg. Dosage for administration of polynucleotides can be from approximately 10⁶ to approximately 10¹² copies of the polynucleotide molecule. Wide variations in needed dosage are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization as is well understood in the art. Agents can be administered once or can be repeatedly administered, as needed.

When an agent to be delivered is a polypeptide, encapsulation of the polypeptide in a suitable delivery vehicle (e.g., polymeric microparticles or implantable devices) may increase the efficiency of delivery, particularly for oral delivery. Alternatively, a nucleic acid containing a nucleotide sequence encoding the polypeptide can be delivered to a subject. Expression of the coding sequence can be directed to any cell in the body of the subject. Expression typically can be directed to cells in the vicinity of the neural cells whose damage it is desired to inhibit. Expression of the coding sequence can be directed to the neural cells themselves. This can be achieved by, for example, the use of polymeric, biodegradable microparticle or microcapsule delivery devices known in the art.

Another way to achieve uptake of a nucleic acid is using liposomes, which can be prepared using standard methods. The vectors can be incorporated alone into these delivery vehicles or co-incorporated with tissue-specific or tumor-specific antibodies. Alternatively, one can prepare a molecular conjugate composed of a plasmid or other vector attached to poly-L-lysine by electrostatic or covalent forces. Poly-L-lysine binds to a ligand that can bind to a receptor on target cells (Cristiano et αl. (1995), J. Mol. Med. 73:479). Alternatively, tissue specific targeting can be achieved by the use of tissue-specific transcriptional regulatory elements (TRE), including those known in the art. Delivery of "naked DNA" (i.e., without a delivery vehicle) to an intramuscular, intradermal, or subcutaneous site is another means by which to achieve *in vivo* expression.

Where antisense oligonucleotides *per se* are administered, they can be suspended in a pharmaceutically-acceptable carrier (e.g., physiological saline) and administered under the same conditions described herein for other agents that reduce with the pathologic action of ATP. Where an expression vector containing a nucleic sequence encoding an antisense oligonucleotide is administered to a subject, expression of the coding sequence can be directed to a neural cell in the body of the subject using any of the cell- or tissue-targeting techniques described herein.

In any of the above methods of reducing neural damage due to the pathologic action of ATP, one or more agents (*e*.*g*., one, two, three, four, five, six, seven, eight, nine, ten, 11, 12, 15, 18, 20, 25, 30, 40, 50, 60, 70, 80, 100, or more) including, for example, P2X receptor antagonists, ATP degrading enzymes, antisense oligonucleotides, siRNAs, drugs, or small molecules (or vectors encoding them), can be used.

### 3. Compositions, kits, and articles of manufacture

Agents useful in the methods provided herein can be used in the manufacture of medicaments for reducing neural damage due to the pathologic action of ATP on neurons. Thus, provided herein are compositions containing a pharmaceutically acceptable carrier and one or more ATP inhibitory agents, *i.e*., agents that inhibit the pathologic action of ATP on neurons. Also provided herein are articles of manufacture that include packaging material, one or more agents that inhibit the pathologic action of ATP on neurons, and written instructions for use of the one or more agents to reduce neural damage due to pathologic action of ATP. Further provided herein are kits containing one or more agents that inhibit the pathologic action of ATP on neurons. A kit can further include, for example, a syringe or other device by which to administer the one or more agents to a subject. A kit also can include a label or instructions for use of the one or more agents to reduce neural damage due to pathologic action of ATP.

The invention will be further described in the following examples, which do not limit the scope of the invention described in the claims.

### EXAMPLES

### Example 1- Materials and Methods

**Pressure controlled incubation:** A free-standing bioreactor chamber was used for incubation of living retinas (e.g., rat retinas) in artificial cerebrospinal fluid (ACFS; Stacy et αl. (2003) J. Comp. Neurol. 456:154-166) under controlled temperature (33 ± 0.5°C; pH 7.4 ± 0.1), and hydrostatic pressure conditions (10-90 mm Hg, resolution 2 mm Hg; Previti, et αl. (2002) IEEE-EMBS Special Topic conference on Molecular, Cellular and Tissue Engineering, 1:157-158). Rat retinas were allowed to stabilize at 12 mm Hg for 5 minutes, and 1 minute pressure increments were applied, reaching a peak value in less than 20 seconds. Consecutive pressure transients were separated by 1 minute at 12 mm Hg. Hydrostatic pressure and pH were regulated by electronically controlled air/CO₂ admission in the incubator chamber.

**Imaging living RGCs:** Experiments were performed on Long Evans rats according to the ARVO and national regulation on animal experimentation. All chemicals were obtained from Sigma (St. Louis, MO) unless otherwise specified.

Adult animals were decapitated and the retinas quickly dissected, flattened onto nitrocellulose filters, and maintained in oxygenated ACSF. Individual RGCs were labeled by shooting 1.3 µm tungsten particles coated with Oregon Green 488 conjugated dextran (Molecular Probes, Inc., Eugene, OR) into the retina using a gene gun (Bio-Rad Laboratories, Hercules, CA; Kettunen, et αl. (2002) J. Neurosci. Methods 119:37-43). Individual RGCs were imaged by placing the retinas in oxygenated ACSF within a custom-made 4 ml observation chamber on the stage of a Zeiss Axioplan fluorescence microscope equipped with a black and white CCD camera (Chroma DTA, Pisa, Italy). Observations were performed at room temperature (25-28°C). A 40x Olympus immersion objective (N.A. 0.80) was used and excitation light was kept below 10% of its maximum intensity. Less than 5 RGCs per retina were analyzed to minimize light exposure. Loss of membrane integrity was assessed by adding 1.5 µM PI to the ACSF for 1 minute. Exposure of phospatidylserine on the outer membrane leaflet was evidenced by a 20 minute incubation in ACSF containing 3 µg/ml Cy3-Annexin V. In some experiments, RGCs were preincubated with oxidized-ATP (oATP) for 2 hours or with Brilliant BlueG (BBG) for 30 minutes prior to imaging.

**Pressure transients *in vivo:*** Adult Long Evans rats were deeply anaesthetized by intraperitoneal injection with Avertine (10 ml/kg body weight, 3.3% tri-bromo-ethanol, 2% tertiary amyl-alcohol in saline) and their right eyes were incannulated with a 30 Gauge needle connected to a pressure apparatus, which was a column of ACSF held at appropriate height. Eye pressure was increased to 50 mm Hg for 2 minutes by switching on the connection between the needle and the column of ACSF. A piezo-resistive pressure sensor (RS Electronics) controlled pressure values and line tightness. This entire procedure was performed under a dissecting microscope to control for eye damage. Pupil dilation was achieved with topical atropine, and the use of a contact lens enabled monitoring of retinal blood vessels while pressure was applied. After pressure application, the animals were returned to their cages for recovery. Retinas were isolated after one hour. Control eyes were incannulated, but no pressure transient was applied. To analyze the effects of blocking eATP *in vivo,* 2 µl apyrase (30 U/ml intraocular concentration) were injected intraocularly 5 minutes before pressure application. To evaluate cell damage, PI labeled cells were imaged across the retinal area in 10 evenly spaced (370 x 250 µm²) sample fields per retina. This dye permits assessment of membrane integrity, as cells with reduced membrane integrity are permeable to and become stained with the dyes. Retina dissection, histological and immunocytochemical treatments for long term analysis were performed as described (Galli-Resta et αl. (1997) J. Neurosci. 17:7831-7838).

To measure ATP levels, 5 µl vitreal body samples of were collected from anaesthetized animals by making a hole in the posterior chamber of the eye with a 30 gauge needle inserted right behind the ora serrata and then positioning in the hole a glass micropipette with a 50 µm tip. The micropipette was connected to a 25 µl Hamilton syringe with a microdriven piston that allowed sample volume control. Samples were collected 90 seconds after the pressure pulse and ATP levels measured by the luciferin-luciferase assay in a luminometer (Wallac Victor 31420, Perkin Elmer, Boston, MA).

**Extracellular electrophysiological recordings:** Experimental procedures for extracellular electrophysiological recordings are as described (Caleo et al. (2003) J. Neurosci. 23(1):287-296.). Briefly, rats aged between P25 and P30 were anesthetized with urethane (20% solution in saline; 0.7 ml/100 g of body weight, i.p.; Sigma) and placed in a stereotaxic frame. Both eyes were fixed by means of adjustable metal rings surrounding the external portion of the eye bulb. Body temperature was continuously monitored and maintained at 37°C by a thermostat-controlled electric blanket. After exposure of the cerebral surface, a glass micropipette (tip resistance, 2MΩ) filled with 3 M NaCl was inserted into the brain at the appropriate stereotaxic coordinates (2.1 mm anterior and 3.3 mm lateral from lambda) and lowered to reach the optic tract which is composed by RGC axons (90% ca. from the controlateral eye, 10% from the ispilateral eye. The first evoked visual activity was usually encountered at a depth of 3.6 mm from the pial surface and had an audible "swish," characteristic of discharges from fibers of the optic tract. Optic tract location was confirmed by histological reconstruction of the electrode track in a few animals, as described (Caleo *et αl., suprα*)*.* Visual stimuli were light flashes of 1.25 seconds generated by a VSG2/5 card (Cambridge Research Systems, Rochester, UK) on a display (Sony Multiscan G500) positioned 20-30 cm in front of the rat's eyes. Stimulus frequency was 0.2 Hz, flash contrast 80%, and mean luminance was 15 cd/m2. Signals were amplified 25,000-fold, bandpass filtered (500-5000Hz), and conveyed to a computer for storage and analysis with a custom made program (based on a National Instrument Card). Multi-unit spikes were discriminated from background by a voltage threshold, that was set between 3.5 and 4.5 times the standard deviation of noise. Responses were averaged over 10 consecutive stimulations. Recovery time was defined as the first time after pressure stimulation when the variable under consideration reached 85% of its value before pressure application.

### Example 2 - Effects of increased pressure on RGCs in vitro

To investigate the mechanisms of pressure-induced RGC damage, rat retinas were isolated, individual living RGCs were labeled, and their somas, dendrites and axons were imaged before and after controlled pressure increments. This procedure enabled examination of the effects of pressure on single neurons in a manner that was independent of the alteration of retinal blood supply that an increase in IOP might cause *in vivo* (Quigley (1999) Prog. Retin. Eye Res. 18:39-57). The focus was on short high pressure transients, which can induce temporary visual impairment (Siliprandi et αl. (1988) Invest. Ophthαlmol. Vis. Sci. 29:558-565).

While 1 hour of incubation at physiological pressure level (12 mm Hg) did not affect any RGCs, 1 minute at 50 mm Hg (1x50) affected 25% of the cells, causing blebbing of the cell soma and of the axonal initial segment within 1 hour. Permeability to PI revealed disruption of membrane integrity in half of the blebbing cells (12% RGC). With higher pressure values or repeated pressure insults the percentage of affected cells increased, and blebbing became more severe and extended to the dendrites. One hour after 7 one-minute pulses at 90 mm Hg (7x90), all but the α-type RGCs displayed blebbing and loss of membrane integrity. A few hours after the 7x90 pressure stimuli, the cell somas appeared shrunken or fragmented (10/10 RGCs), and phosphatidylserine exposure was observed on the external membrane, as revealed by Annexin V binding (10/10 RGCs), indicating activation of a death process (Wyllie et αl. (1980) Int. Rev. Cytol. 68:251-306). This progressive degeneration was not observed after a 1x50 stimulus. Pressure induced RGC damage is quantified in Figure 1. The Fisher 2x2 two-tail test showed that the damaging effects of pressure are statistically significant (1x50: P < 0.001; 7x90: P < 10⁻¹¹) as are the protective effects of blocking eATP signaling (7x90 + eATP blockade: P < 10⁻¹¹ ; 1x50 + eATP blockade: P < 10⁻³).

### Example 3 - Effects of ATP reducing αgents on pressure-induced RGC dαmαge in vitro

The effects of eATP can be mediated by the P2X₇ receptors (Di Virgilio et αl. (1998) Cell Death Differ. 5:191-199; and North (2002) Physiol. Rev. 82:1013-1067. Thus, experiments were conducted to determine whether eATP participates in pressure-induced RGC damage.

RGC were incubated with the following agents: 30 U/ml apyrase (an enzyme that degrades extracellular ATP; North, *suprα*)*;* 300 µM oATP (an irreversible blocker of P2X receptors; North, *suprα*)*;* or 0.5 µM BBG (a selective and reversible inhibitor of rat P2X₇ receptors; North, *suprα*)*.* Each of these agents prevented all signs of pressure induced RGC damage, even under the most adverse pressure conditions tested (7x90; Figure 1). For example, no blebbing or permeability to PI was observed when pressure was applied in the presence of apyrase. Blocking eATP also protected RGCs from damage caused by the 1 minute pulse at 50 mm Hg (1x50).

Incubation of isolated rat retinas in 1 mM ATP induced the same effects obtained with repeated pressure increments, causing cell blebbing and uptake of PI in all the RGCs tested (35/35 tested) within 1 hour, except in α-type RGCs (0/15). The same effects were obtained applying the potent P2X₇ receptor agonist 2',3'-benzoyl-4-benzoyl-ATP (BzATP; 0.1mM; 10/10 non-α-RGCs). Two hours after ATP application, phosphatidylserine exposure was observed on the external membrane, as revealed by Annexin V binding (7/7 RGCs), and cell soma shrinkage or fragmentation also was observed (10/10 RGCs). Both of these phenomena are typical indicators of the activation of a cell death process (Wyllie *et αl., supra;* and Reutelingsperger et al. (2002 J. Immunol. Methods 265:123-132). ATP-induced RGC damage was totally prevented by a 2 hour preincubation in ACSF containing either 300 µM oATP (0/20 non-α-RGCs) or 0.5 µM BBG (0/15 non-α-RGCS). Thus, degrading eATP or blocking the cytotoxic P2X₇ receptors for ATP prevented pressure damage in isolated retinas. Conversely, direct ATP application was shown to mimic RGC damage induced by high pressure.

### Example 4 - Effects of increased pressure on RGCs in vivo

To test whether pressure has similar effects *in vivo,* a brief intraocular pressure transient (2 minutes at 50 mm Hg) was applied to anaesthetised adult rats, while visually monitoring the retinal blood vessels to ensure that they were unaffected by pressure. One hour after the pressure pulse, retinas were isolated in oxygenated ACSF to test for RGC damage. It was observed that 10% of the cells in the ganglion cell layer stained with PI, a clear sign of cell injury. This percentage of PI labeled cells was very similar to that observed *in vitro* after a single pressure pulse at 50 mm Hg. Ninety seconds after the pressure pulse, a 5-fold increase in the ATP content was observed in the vitreal body of treated rats with respect to control animals (control 0.37 ± 0.15 µM, N=8; treated 2.1 ± 0.6 µM N=6; P<0.01 t-test). Degrading extracellular ATP with intraocular injection of apyrase (30 U/ml intraocularly) 5 minutes before pressure application totally prevented pressure-induced RGC damage *in vivo* (Figure 2; P < 10⁻⁵). Thus, brief pressure transients caused ATP release and damage RGCs *in vivo,* and RGC damage was completely prevented by degrading endogenous extracellular ATP.

To investigate whether pressure-induced damage was irreversible *in vivo,* animals were allowed to survive for 5 days after the pressure pulse, since at this time cell loss is apparent in classical models of RGC injury such as optic nerve transection (Kermer et αl. (1999) FEBS Lett. 453:361-364). Retinas exposed to pressure did not show any significant reduction in the number of RGCs or picnotic cells, nor did they exhibit typical early signs of damage such as the activation of caspase 3, c-fos or phospho-jun. This suggested that RGCs might recover from damage induced by single pressure transients in *vivo.*

To test this directly, retinas were isolated after application of a pressure pulse in *vivo,* and two different dyes that can reveal loss of membrane integrity (YOYO-pro and PI) were added either simultaneously or sequentially. When the dyes were applied simultaneously, all cells permeable to YOYO were also permeable to PI (200/200 cells). By applying YOYO 1 hour after pressure followed by PI 2 hours after pressure, it was possible to assess whether any cells had lost membrane integrity at 1 hour but had recovered it at 2 hours, as such cells would incorporate YOYO-pro but not PI. Many cells were labeled with both dyes. In addition, sequential addition of the dyes revealed neurons that had lost membrane integrity 1 hour after pressure application, as shown by YOYO-pro labeling, but had recovered membrane integrity by 2 hours and thus were impermeable to PI. YOYO-pro labeling typically showed that these cells had little cytoplasmic shrinkage and little nuclear staining, indicating that even at 1 hour they had undergone limited damage. At this time, recovering cells represented 30 ± 20% of the damaged cells.

### Example 5 - Effects of intrαoculαr injection of αpyrαse

Experiments were conducted to determine (1) whether intraocular injection of apyrase had any deleterious effects on RGC firing activity and light response, and (2) whether intraocular injection of apyrase had any effect on RGC light response recovery following a transient IOP elevation *in vivo.* Multi-unit spike activity evoked by a light flash was recorded in the optic tract at five minute intervals before and after an IOP pulse at 90 mmHg for 1 minute. Typically, in multiunit recordings, both the onset and the offset of a light flash evoke significant increments in firing rate, as exemplified in Figures 3a, 3b, and 4a. After a single IOP spike this response to light is temporarily reduced or almost abolished (Figure 4b), then gradually recovers (Figure 4c), in line with what observed in single RGC recording (Troy and Shou (2002) Prog. Retin. Eye Res. 21(3):263-302). RGC activity was elicited by both light onset and offset, giving rise to a double peak in the PSTH (Figure 4c). The example illustrates a case of strong reduction of RGC activity soon after IOP. Quantifying response as the average firing rate in the interval 0-2 seconds after flash onset, the average maximal response decrement was 80 ± 16 %; N = 15, and the average time needed to recover at least 85% of the initial response was 32 ± 4 minutes after the IOP spike. Two typical time courses for response recovery in normal animals are illustrated in Figure 5a, while two examples of response recovery after IOP spike in apyrase-treated animals are illustrated in Figure 5b. Apyrase treatment (30 U/ml intraocularly) 1-3 hours before IOP increase shortened the average response recovery-time to 11.2 ± 2.6 minutes (N = 8), while not significantly altering spike activity before pressure application. The difference in recovery time between normal and apyrase treated animals was statistically significant (P < 0.001). Thus, reducing eATP levels in the eye with apyrase injection does not negatively affect RGC light responses, but rather improves their recovery following IOP transient.

Taken together, these studies showed that endogenous extracellular ATP mediates RGC damage induced by brief pressure increments *in vitro* and *in vivo. In vitro* studies allowed monitoring the effects of pressure at the single cell level, and showed that neural injury after single pressure pulses occurred independently of effects on blood supply that pressure might cause *in vivo.* Parallel experiments showed that short pressure transients caused ATP release *in vivo* and resulted in damage to RGCs. Pressure effects were mimicked by direct application of ATP to isolated retinas, and conversely, blocking eATP signaling, either by degrading endogenous extracellular ATP or blocking the activation of the P2X₇ receptors for ATP, totally prevented pressure-induced RGC damage both in *vitro* and *in vivo.*

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A composition or kit for use in inhibiting neuronal damage in a mammal due to increased extracellular pressure, the composition comprising one or more agents that are inhibitors of the pathologic action of ATP on a neurons, or the kit comprising two or more such agents.

2. A composition or kit as described in claim 1, for the use described therein; wherein at least one of said agents is a P2X receptor antagonist, an agent that degrades ATP, or an agent that reduces or prevents expression of a protein involved in ATP synthesis or signaling.

3. A composition or kit as described in any preceding claim for the use described therein; wherein at least one of said agents is an antibody or antibody fragment that binds specifically to a P2X receptor or to another molecule involved in ATP cell signaling via said receptor; Brilliant BlueG; oxidized-ATP (oATP); pyridoxalphosphate-6-azophenyl-2'.4'- disulphonic acid.4Na (PPDS); suramine; 1-[N,O-bis(5-isoquinolinesulfonyl)-N- methyl-L-tyrosyl)-4-phenylpiperazine (KN-62); 5-(N,N-hexarnethlyiene)amiloride (HMA); 5-{[3"-diphenylether (1',2',3',4'- tetrahydronaphthalen-1-yl)amino)carbonyl}benzene-1, 2, 4-tricarboxylic acid; 2',3'-*O*-(4-benzoylbenzoyl)-ATP (BzATP); tetramethylpyrazine (TMP); 2',3 '-*O*-2,4,6-trinitrophenyl-ATP (TNP-ATP); a nucleic acid aptamer; an antisense nucleic acid; an interfering RNA; an ecto-nucleotidase (e.g. an apyrase), a hexokinase, or another enzyme that degrades extracellular ATP.

4. A composition or kit as described in any preceding claim, for the use described therein; wherein at least one of said agents is apyrase, Brilliant BlueG or oxidized ATP.

5. A composition or kit as described in any preceding claim, for the use described therein; wherein the increased extracellular pressure is:
(i) spontaneous, traumatic, pathologic, or associated with surgery; or
(ii) intraocular, intracranial, or in the spinal fluid.

6. A composition or kit as described in any preceding claim, for use in one or more of the following: treating glaucoma, treating neural trauma, treating brain edema, treating hydrocephaly, opthalmic surgery or intracranial surgery

7. A composition or kit as described in any preceding claim, for the use described therein; wherein the mammal is a human.

8. A composition or kit as described in any preceding claim, for the use described therein; wherein the neuronal damage that is inhibited is in respect of a retinal neuron (e.g. a ganglion cell), a cortical neuron, a hippocampal neuron, a basal ganglia neuron, or a spinal cord neuron.

9. A composition comprising a pharmaceutically acceptable carrier and one or more agents that can inhibit the pathologic action of ATP on neurons by inhibiting neuronal damage due to increased extracellular pressure.

10. An article of manufacture (e.g. a kit) comprising packaging material, one or more agents that can inhibit the pathologic action of ATP on neurons by inhibiting neuronal damage due to increased extracellular pressure, and written instructions for utilising said one or more agents in a use as described in any of claims 1 to 8.

11. A kit comprising two or more agents that can inhibit the pathologic action of ATP on neurons due to increased extracellular pressure.

12. A composition according to claim 9, an article of manufacture according to claim 10, or a kit according to claim 11; wherein at least one of the one or more agents is as described in any of claims 2 to 4.

13. Two or more agents that inhibit the pathologic action of ATP on neurons for simultaneous sequential or combined use in inhibiting neuronal damage in a mammal due to increased extracellular pressure.

14. Two or more agents as described in claim 13 for use as described therein; wherein at least one of the two or more agents is as described in any of claims 2 to 4.

15. Two or more agents as described in claim 13 for use as described therein; wherein said agents are as described in any of claims 2 to 4.
